# EUROPEAN PATENT APPLICATION

(11) **EP 1 792 996 A1**
(43) Date of publication of application: **06.06.2007**
(21) Application number: 05026255.9
(22) Date of filing: 01.12.2005
(51) Int. Cl.: C12N 15/86, A61K 39/215

(54) **Nucleic acid sequences encoding vaccines against Porcine reproductive and respiratory syndrome virus (PRRSV)**

(71) Applicant: Consejo Superior de Investigaciones Cientificas, 28006 Madrid (ES); Fort Dodge Veterinaria, S.A., 17813 Vall de Bianya Gerona (ES)
(72) Inventor: Enjuanes Sanches, Luis, 28109 Madrid (ES); Zuniga Lucas, Sonia, 28760 Madrid (ES); Sola Gurpegui, Isabel, 28700 Madrid (ES); Plana Durán, Joan, 17811 Santa Pau, Girona (ES)
(74) Representative: UEXKÜLL & STOLBERG

(57) **Abstract**

The present invention relates to nucleic acids comprising:
(a) sequences of a replication competent transmissible gastroenteritis virus (TGEV), which sequences encode a TGEV replicase under the control of expression regulatory sequences, wherein the replicase is expressed in a host cell and will initiate replication of the nucleic acid and thus increase the number of nucleic acids in the cell; and
(b) a sequence encoding at least one neutralizing epitope of ORF5 of porcine reproductive and respiratory syndrome virus (PRRSV), which sequence includes a disulfide bridge forming residue; and
(c) a sequence encoding at least one further polypeptide capable of increasing an immune response against PRRSV.

The present invention further relates to vectors, virus particles and host cells comprising these nucleic acids as well as their use for the preparation of vaccines, specifically for the preparation of vaccines.

## Description

The present invention is directed to nucleic acids comprising sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell. The nucleic acids of the present invention further encode neutralizing epitopes of PRRSV proteins and one or more polypeptides capable of increasing an immune response against PRRSV. The present invention is further directed to the use of these nucleic acids for the preparation of pharmaceutical compositions in general and specifically for the preparation of vaccines.

### TECHNICAL BACKRGOUND

Therapy approaches that involve the insertion of a functional gene into a cell to achieve a therapeutic effect are also referred to as gene therapy approaches, as the gene serves as a drug. Gene therapy is a technique primarily for correcting defective genes responsible for disease development.

A carrier molecule also referred to as a vector is used to deliver the therapeutic gene to the patient's target cells. Currently, the most common vector is a virus that has been genetically altered to carry human or animal genes. Viruses have evolved a way of encapsulating and delivering their genes to human or animal cells in a pathogenic manner. Scientists have taken advantage of this capability and manipulate the virus genome to remove disease-causing genes and insert therapeutic genes.

These viral vectors were used for expressing heterologous genes that cause an immunogenic response in the subject receiving the vector and thus immunize that subject. In that case the viral vector serves as a vaccine.

Transmissible gastroenteritis virus is a member of the family of coronaviruses. Coronaviruses are ssRNA(+) viruses which have the largest genome so far found in RNA viruses with a length between 25 and 31 kilobases kb (see Siddell S.G. 1995, The Coronaviridae). When a coronavirus infects a cell, the genomic RNA (gRNA) replicates in the cytoplasm and a set of subgenomic RNAs (sgRNA) of positive and negative polarity is produced (Sethna et al., 1989; Sawicki & Sawicki, J.Virol., 1990; and Van der Most and Spaan, The Coronaviridae).

Due to the fact that the coronaviruses replicate in the cytoplasm, use of coronaviruses as a vector for gene therapy and vaccination has been suggested (Enjuanes et al., 2003). Specifically, defective interfering (DI) genomes of coronaviruses were produced. These DI genomes are deletion mutants which require the presence of a complementing or helper virus for replication and/or transcription (see Chang et al., 1994; WO97/34008; Spanish patent application P9600620; Izeta et al., 1999; and Sanchez et al., 1999).

The entire genome of a coronavirus was cloned in the form of an infectious cDNA (Almazan et al., 2000 and WO01/39797). The cloning of the entire genome allowed the preparation of infectious vectors containing heterologous sequences suitable for expression of large proteins in a host cell.

The potential of the cloned viral genome for expression of heterologous sequences was reviewed in Enjuanes et al., 2003.

Using the cloned virus the structure of the genome and relevance of the coronaviral genes for infection were assessed by preparing deletion mutants. It was found that genes 3a, 3b and 7 are non-essential for replication of the viral nucleic acid and that absence of the genes reduces pathogenicity of the virus (Ortego et al., 2002 and 2003; Sola et al., 2003).

Porcine reproductive and respiratory syndrome virus (PRRSV) was first identified in 1991 as the causative agent of a new disease in pigs (Wensvoort *et al.,* 1991). Since then, PRRSV has become one of the leading causes of economic losses in swine operations worldwide and it is currently accepted as the most important infectious disease of swine, causing reproductive failure in adult animals and severe pneumonia in neonatal pigs. PRRSV is a member of *Arteriviridae* family that belongs to *Nidovirales* order. Two genotypes (American and European) are recognized (Murtaugh *et al.,* 1995). PRRSV is an enveloped virus with a single-stranded positive-sense RNA genome of 14.5 Kb. The 5' two-thirds of the genome encode the replicase polyproteins (pp1a and pp1ab), the rest of the genomic RNA encodes three minor membrane-associated glycoproteins (Gp 2, Gp3 and Gp4) and the three mayor structural proteins (Gp5, M and N).

Pigs are generally infected with PRRSV by following exposure of the mucosal surface or the respiratory tract to the virus. A hallmark of the swine antibody response against PRRSV is the abundant non-neutralizing antibodies detected early in the infection, followed by a low neutralizing antibody titer that appears more than 3 weeks after infection. Experimental data showing the importance of neutralizing antibodies in protection against PRRSV infection have been collected in the last years (Lopez & Osorio, 2004).

Immune response to PRRSV is poorly understood but, in spite of this, some vaccines are being commercialized. Current vaccines against PRRSV have several drawbacks. Modified live vaccines protect against challenge with homologous isolates, but generally have a limited effect against challenge with heterologous viruses (Meng, 2000). Furthermore, live vaccines provide partial protection against clinical disease but did not prevent infection (Osorio *et al*., 1998) and, more importantly, they can revert to virulence (Botner *et al*., 1997, Nielsen *et al.,* 2001). As the attenuated vaccines induce an immune response resembling that induced by PRRSV natural infection, they do not induce high levels of neutralizing antibodies. Killed PRRSV vaccines, on the other hand, have proved to be less effective in prevention of both infection and disease (Ostrowski *et al.,* 2002).

The problem underlying the present invention thus resides in providing effective vaccine vectors with good safety and immunogenicity against PRRSV.

### SUMMARY OF THE INVENTION

According to a first aspect of the present invention a nucleic acid is provided which comprises:
(a) sequences of a replication competent transmissible gastroenteritis virus (TGEV), which sequences encode a TGEV replicase under the control of expression regulatory sequences, wherein the replicase is expressed in a host cell and will initiate replication of the nucleic acid and thus increase the number of nucleic acids in the cell; and
(b) a sequence encoding at least one neutralizing epitope of ORF5 of porcine reproductive and respiratory syndrome virus (PRRSV), which sequence includes a disulfide bridge forming residue; and
(c) a sequence encoding at least one further polypeptide capable of increasing an immune response against PRRSV.

According to a second aspect of the present invention a nucleic acid is provided which comprises:
(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and
(b) sequences encoding multimers of one or several neutralizing epitopes of ORF5 of PRRSV and at least one further polypeptide capable of increasing an immune response against PRRSV.

In a preferred embodiment the neutralizing epitope is defined by the amino acid sequence TYQYIYN (SEQ ID NO: 10).

The replication competent TGEV sequences need not but may further encode other TGEV proteins. The TGEV sequences may thus encode a fully infectious TGEV virus and the sequences of the neutralizing epitope or multimers of neutralizing epitopes just comprise a replication competent nucleic acid. The present invention further relates to vectors comprising a respective nucleic acid and host cells comprising the vector. The host cells may be capable of complementing TGEV genes that may have been deleted from the nucleic acids of the present invention. The host cell thus may be a packaging cell line or may contain a helper virus expressing TGEV genes, so that a TGEV virus particle is formed that comprises the sequences of at least one neutralizing epitope of PRRSV. Virus particles obtained by association of the TGEV coat proteins with the replication competent but non-infectious nucleic acids of the present invention are an especially preferred embodiment of the present invention (corresponding virus particles have also been referred to as pseudoviruses).

Finally, the present invention is also directed to the medical use of the nucleic acids, the virus vectors and the host cells specifically to the use as a vaccine for treating or protecting animals, such as a swine against infectious diseases. The vaccine can thus be administered to an animal to reduce or eliminate the symptoms of a subsequent infection of a wild-type virus.

### DETAILED DESCRIPTION OF THE INVENTION

The present invention is thus directed to a nucleic acid comprising:
(a) sequences of a replication competent transmissible gastroenteritis virus (TGEV), which sequences encode a TGEV replicase under the control of expression regulatory sequences, wherein the replicase is expressed in a host cell and will initiate replication of the nucleic acid and thus increase the number of nucleic acids in the cell; and
(b) a sequence encoding at least one neutralizing epitope of ORF5 of porcine reproductive and respiratory syndrome virus (PRRSV), which sequence includes a disulfide bridge forming residue; and
(c) a sequence encoding at least one further polypeptide capable of increasing an immune response against PRRSV.

The present inventors have surprisingly found that expression of at least one neutralizing epitope or multimers of such an epitope of ORF5 of PRRSV and at least one further polypeptide capable of increasing an immune response against PRRSV in the context of the TGEV vector backbone leads to an efficient and very safe vaccine against PRRSV.

The use of the TGEV-based vector will allow the delivery of the epitopes to the desired tissues with high antigen expression levels and the vector will be engineered to generate a safe vaccine.

The TGEV-based vector can exhibit a modified vector-tropism. In one embodiment of the invention the vector is a respiratory tract directed vector. This is achieved by using the spike (S) gene from a respiratory virus. In an alternative embodiment the vector is an enteric tract directed vector. This is achieved by using the S gene from an enteric strain of TGEV.

In a preferred embodiment a modified S gene is used (SEQ ID No:1) which provides enteric tropism and is very stable upon passage in swine testis (ST) cells in culture.

In its broadest aspect the nucleic acid of the present invention is characterized as a nucleic acid encoding replication competent TGEV sequences that means sequences encoding a TGEV replicase under the control of expression regulatory sequences so that expression of the replicase in a cell containing the nucleic acid will initiate replication of the nucleic acid and thus increase the number of nucleic acids in the cell. Once a cell is infected by the nucleic acids of the present invention, the gene for the replicase will be expressed and the nucleic acid will be replicated. The more copies of the nucleic acid are present in the cell the more epitopes will be expressed.

In accordance with the present invention "neutralizing epitope" means epitopes which are involved in virus neutralization. Antibodies which recognize Gp5 (encoded by ORF5) of PRRSV neutralize PRRSV more effectively than the ones specific for other viral proteins. It is to be understood that the nucleic acid of the invention encodes at least a neutralizing epitope of ORF5 of PRRSV but can also encode more neutralizing epitopes or a larger part of ORF5, for example the whole Gp5 protein.

In a preferred embodiment the neutralizing epitope is defined by the amino acid sequence TYQYIYN (SEQ ID NO: 10).

In one embodiment the nucleic acids of the invention encode at least one neutralizing epitope of ORF5 of PRRSV and a dislulfide bridge forming residue which can be used to connect a further polypeptide to said neutralizing epitope. For example, an epitope of ORF6, which encodes the M protein of PRRSV, or the complete M protein can be coupled to the epitope of ORF 5 via a disulfide bridge.

In another embodiment the nucleic acids according to the invention encode multimers of neutralizing epitopes of ORF5. A "multimer" comprises at least 2 copys of one epitope. A "multimer" may comprise repetitions of Gp5 or other PRRSV derived protein sequences inducing neutralizing antibodies to PRRSV. This definition includes synthetic protein domains (or peptides) derived from PRRSV Gp5 or other PRRSV proteins with a modified sequence by point mutagenesis leading to an immunogenic structure providing higher protection because of an enhanced neutralizing response or removal of decoy epitopes.

Typically, a nucleic acid will encode for multimers comprising 2 to 5 repetitions of the sequence encoding the neutralizing epitope of ORF5 of PRRSV. These multimers may be connected by a nucleic acid sequence encoding a flexible linker of 2 to 8, preferably 3 to 6 amino acids. These "linker residues" will improve the flexibility of the epitopes. A specifically preferred linker is the sequence Gly-Gly-Pro-Gly-Gly (SEQ ID NO:15).

In a further embodiment the nucleic acids encode neutralizing epitopes of ORF5 that have been modified to knock out glycosylation sites. In a preferred embodiment such glycosylation sites are amino acid 46 and/or 53 of Gp5 from PRRSV Olot 91 strain.

The glycosylation of the Gp5 protein epitopes can interfere with the induction of antibodies resulting in a decreased immune response. Therefore, the epitopes encoded by the nucleic acids of the invention are able to provide increased stimulatory effects of the immune response compared to naturally occurring epitopes.

In a preferred embodiment of the invention the nucleic acid encodes at least one neutralizing epitope of ORF5 and ORF6 but no further PRRSV polypeptides. This results in a very effective vaccine against PRRSV. In another preferred embodiment the nucleic acid encodes whole ORF5 and ORF6 but no further PRRSV polypetide.

In a further preferred embodiment the nucleic acid encodes one neutralizing epitope of ORF5 and whole ORF6 but no further PRRSV polypetide. In yet another preferred embodiment the neutralizing epitope of ORF5 is defined by SEQ ID NO:10.

Other PRRSV derived proteins that could be expressed to induce a protective immune response are the glycoproteins Gp2, Gp3 and Gp4. It has been shown that these proteins are incorporated into virions as a multimeric complex and are essential for virus infectivity. Therefore, in a further embodiment of the invention the nucleic acids encode at least one neutralizing epitope of Gp5 and at least one neutralizing epitope of Gp2, Gp3 or Gp4. In a preferred embodiment the nucleic acids encode a neutralizing epitope of Gp5 and Gp2. In another embodiment the nucleic acids encode a neutralizing epitope of Gp5 and Gp3 and in yet another embodiment the nucleic acids encode a neutralizing epitope of Gp5 and Gp4. Further embodiments comprise the combinations Gp5 with Gp2 and Gp3, Gp5 with Gp2 and Gp4 or Gp5 with Gp3 and Gp4.

In a further embodiment of the invention the nucleic acid comprises a nucleic acid comprising:
(a) sequences of a replication competent transmissible gastroenteritis virus (TGEV), which sequences encode a TGEV replicase under the control of expression regulatory sequences, wherein the replicase is expressed in a host cell and will initiate replication of the nucleic acid and thus increase the number of nucleic acids in the cell;
(b) residues 118 to 138 of SEQ ID NO:2 or a sequence having a similarity of 90% to these residues under the control of an expression regulatory sequence; and
(c) SEQ ID NO:3 or a sequence having a similarity of 90% to SEQ ID NO:3 under the control of an expression regulatory sequence; wherein
the sequences of (a) and (b) and (c) are each under the control of a different expression regulatory sequence.

In one embodiment the nucleic acid characterized by the specific residues of SEQ ID NO:2 and SEQ ID NO:3 further comprises nucleic acid sequence SEQ ID NO:12, which encodes the lysosomal targeting signal of lysososmal integral membrane protein-II (LIMP-II) or a sequence having a similarity of 90% to SEQ ID NO:12. The sequence encoding the LIMP-II protein is preferably fused to the SEQ ID NO:3 so that the nucleic acid is capable of expressing a fusion protein which comprises the ORF6 sequence of PRRSV and the LIMP-II sequence. In one aspect of this embodiment of the present invention, the nucleic acid may contain sequences encoding a multimer of the epitope of ORF5 as defined above (i.e. 2 to 5 repetitions of the sequence having the residues 118 to 138 of SEQ ID NO:2 optionally interrupted by a linker of 2 to 8, preferably 3 to 6 amino acids) and SEQ ID NO:3, but the nucleic acid does not contain any further sequences derived from PRRSV, i.e. no other sequences having a homology of 80%, preferably 90 or 95%, to the known sequence of the strain PRRSV Olot 91.

The use of virulent, attenuated, or recombinant antigens of PRRSV may lead to a delay in the induction of virus neutralizing antibodies. In one embodiment of the invention the nucleic acids encode polypeptides which improve antigen presentation. In a preferred embodiment the lysosomal targeting signal of lysosomal integral membrane protein-II (LIMP-II) is used for such purpose. In a further embodiment the whole LIMP-II protein is used and other embodiments comprise any fragment of LIMP-II containing the lysosomal targeting signal. In a more preferred embodiment a fusion protein comprising the lysosomal targeting signal of LIMP-II and ORF5 and/or ORF6, or at least one neutralizing epitope thereof, is encoded by the nucleic acids of the invention, wherein the lysosomal targeting signal of LIMP-II is either fused to the ORF5 or ORF6 and in the case that both, ORF5 and ORF6, are present, the lysosomal targeting signal of LIMP-II is fused to one of them while ORF5 and ORF6, or the neutralizing epitopes thereof, are connected by a disulfide bridge.

In a further embodiment the polypeptide capable of increasing an immune response against PRRSV is an interleukin. Such interleukins are for example IL-2, IL-10 or IL-12. In a preferred embodiment it is interleukin 12 (IL-12) and in a most preferred embodiment it is only the p35 subunit of IL-12.

The replication competent TGEV vector may be infectious or not. A nucleic acid that contains at least all sequences necessary for replication of the nucleic acid, produces one or several coat proteins and associates with the coat proteins to a viral particle that will allow infection of other cells is referred to as an infectious nucleic acid in accordance with the present invention.

In an especially preferred aspect, the present invention provides a virus particle that comprises the above nucleic acid and at least one TGEV coat protein. The virus particle may comprise more than one and even all TGEV coat proteins. A corresponding virus particle will be capable of entering a host cell by way of infection. However, the nucleic acid of such a virus particle may still be infectious or non-infectious, as it need not encode all of the TGEV coat proteins necessary to produce a virus particle. If the nucleic acid is a non-infectious nucleic acid in the sense of the present application, the virus particle is prepared using a packaging host cell or a helper virus that complements the TGEV genes. The use of packaging host cells or helper viruses for obtaining virus particles comprising an incomplete genome of a virus is well known in the art. This way of proceeding has specific advantages, as the virus particle is per se infectious (i.e. can infect a cell once) but the nucleic acid is not capable of producing further infectious virus particles. In other words, the sequences derived from TGEV do not encode proteins that will be capable of associating with the nucleic acid to form a new virus particle. These virus particles thus are extremely safe and still provide a high immunogenic response against the epitopes expressed by the nucleic acids.

According to an alternative embodiment of the present invention the TGEV infectious viral particles obtainable from the association of TGEV proteins and the nucleic acid sequences are attenuated viral particles. This has the advantage that the subject to be treated using the nucleic acids of the present invention will be vaccinated at the same time against TGEV and against PRRSV.

The nucleic acids of the present invention may comprise sequences encoding all proteins of TGEV. Alternatively, the nucleic acids may comprise sequences only encoding the TGEV proteins needed for a replication competent TGEV vector. The nucleic thus preferably encodes the TGEV replicase. According to an especially preferred embodiment, the nucleic acid encodes a replication competent but non-infectious TGEV vector that comprises sequences encoding the TGEV replicase and the TGEV N protein and none of the other TGEV proteins. This vector has the specific advantage that the TGEV vector will be highly amplified in the host cell and thus produce large amounts of the epitopes. At the same time this vector is extremely safe as it is non-infectious.

The term "nucleic acids encoding TGEV proteins" is used herein to refer to nucleic acid sequences as disclosed in Penzes et al., 2001 or nucleic acid sequences having a similarity of at least 60%, preferably at least 75% and most preferably at least 95% to these sequences. For example specific alternative sequences may be used to differentiate between TGEV vaccinated animals and TGEV infected animals (as outlined in more detail below). In the TGEV based vector exemplified in the present application corresponding nucleotide substitutions have been introduced using RT-PCR at positions 6752, 18997, 20460, and 21369, respectively. Especially nucleic acid sequences encoding the TGEV replicase, N protein, M protein, E protein or S protein of TGEV as used herein means nucleic acid sequences as disclosed in Penzes et al., 2001 (with or without the amendments mentioned above). It is of course also possible to use other TGEV strains or to include further deletions, substitutions, insertions or additions into the nucleic acid sequence. According to a further aspect the TGEV sequences thus differ from the sequences disclosed in Penzes et al. but still have a similarity of at least 60%, preferably at least 75% and most preferably at least 95% to these sequences.

The term "nucleic acids encoding PRRSV proteins" is used herein to refer to PRRSV wildtype nucleic acid sequences or nucleic acid sequences having a similarity of at least 60%, preferably at least 75% and most preferably at least 95% to these sequences.

For the purposes of the present application sequence similarity is determined using the ClustalW computer program available from the European Bioinformatics Institute (EBI), unless otherwise stated.

The infectious TGEV vector need not contain genes 3a, 3b and 7, as these are known to be non-essential. The proteins encoded by genes 3a, 3b and 7 of TGEV may modulate the immune response against TGEV and where it is desirable to modulate TGEV interaction with the host, these genes may be maintained in the TGEV vector.

The protein coding sequences within the nucleic acids of the present invention are preferably linked to sequences controlling the expression of these genes in the host cells or organisms. The genes encoding the epitopes or further polypeptides may for example be flanked by transcription regulatory sequences (TRS) and/or internal ribosome entry site (IRES) sequences to increase transcription and/or translation of the protein coding sequences. Respective TRS and IRES sequences are well known in the art.

The nucleic acids of the present invention may be in the form of DNA or RNA. Within the scope of the present invention specifically recombinant RNA molecules are encompassed which are encoded by one of the above nucleic acids.

According to a further aspect the present invention is directed towards vectors comprising one of the above nucleic acids. The vector can be a cDNA vector and preferably is a BAC derived vector, such as BAC-TGEV^{FL}. The vector is preferably capable of replicating the nucleic acid within a specific host cell or a number of host cells.

Host cells, which comprise a vector comprising one of the above nucleic acids are a further subject of the present invention.

The cell may be a bacterial cell, a yeast cell, an insect cell, an animal cell or a human cell. According to a preferred embodiment the cell is a porcine swine testis cell line, such as the cell line deposited under ATCC CRL1746.

The present invention is further directed to polypeptides comprising the neutralizing epitopes of ORF5 and ORF6. In one embodiment such a polypeptide consists of the neutralizing epitope of ORF5 and ORF6 and is encoded by SEQ ID NO: 13 or a sequence having a similarity of 90% to SEQ ID NO:13. In another embodiment such a polypeptide comprises the neutralizing epitopes of ORF5 and ORF6 and additionally the lysosomal targeting sequence of LIMP-II. In a preferred embodiment such a polypeptide consists of the neutralizing epitope of ORF5 and ORF6 and the lysosomal targeting sequence of LIMP-II and is encoded by SEQ ID NO: 14 or a sequence having a similarity of 90% to SEQ ID NO:14.

The present invention further is directed to pharmaceutical compositions comprising one of the nucleic acids, viral RNAs, polypeptides or vectors of the present invention or a host cell as described above. The pharmaceutical composition may further comprise a pharmaceutically acceptable carrier, excipient and/or adjuvants.

In an especially preferred embodiment the present invention relates to vaccines capable of protecting an animal against the disease caused by an infectious virus comprising a nucleic acid, a viral RNA, a vector, a polypeptide or a host cell of the present invention. The vaccine may also comprise pharmaceutically acceptable carriers, excipients and/or adjuvants.

Adjuvants and carriers suitable for administering genetic vaccines and immunogens via the mucosal route are known in the art. Conventional carriers and adjuvants are for example reviewed in Kiyono et al 1996. The addition of chemokines that are used to modulate immune responses are also encompassed by the present invention. Respective compounds and their medical use has been reviewed in Toka et al. 2004. It is specifically advantagous to use one of granulocyte/macrophage colony-stimulating factor, interleukin-2 (IL-2), IL-12, IL-18. Combinatorial approaches utilizing several cytokines and chemokines might also be applied. In addition, as more is discovered regarding the requirements for memory development of T cells, boosters involving key cytokines such as IL-15 and IL-23 may prove beneficial to long-term maintenance of the memory pool.

The vaccine is preferably suitable for treating a mammal, for example a swine. The vaccination of sows is especially preferred.

In accordance with the present invention vaccines are provided, which are preferably capable of inducing both a systemic immune response and a mucosal immune response against PRRSV and/or TGEV.

The vaccine may further be a multivalent vaccine which is capable to provide protection against one or several other pig pathogens. The multivalent vaccine thus may further comprise antigens derived from other viral and/or bacterial pathogens and/or proteins which will provide immunity against pathogens. Examples antigens from further viral pathogens comprise antigens derived from one of Swine Influenza Viruses, Porcine Parvovirus, Porcine Circovirus Type 2, Classical Swine Fever, African Swine Fever, Foot-and-Mouth Disease, Pseudo-Rabies Virus, Porcine Circovirus Type 1, Porcine Adenoviruses, Porcine Enteroviruses, Porcine Respiratory Coronavirus, Porcine Rotavirus, Encephalomyocarditis Virus, Porcine Epidemic Diarrhea Virus, Blue Eye Disease Viruses, Hepatitis E Virus and/or West Nile Virus, Nipah virus. The use of antigens derived from one or several of Swine Influenza Viruses, Porcine Parvovirus, Porcine Circovirus Type 2, Classical Swine Fever, African Swine Fever and/or Foot-and-Mouth Disease is specifically preferred.

Examples antigens from bacterial pathogens comprise antigens derived from one of Mycoplasma hyopneumoniae, Actinobacillus pleuropneumoniae, Actinobacillus suis, Haemophilus parasuis, Pasteurella multocida type A (toxins), Pasteurella multocida type D (toxins), Bordetella bronchiseptica, Isospora suis, Brachyspira hyodysenteriae, Brachyspira pilosicoli, Lawsonia intracellularis, Erysipelothrix rhusiopathiae, Eschericia coli, Salmonella enterica, Mycoplasma hyorinis, Streptococcus suis, Clostridium perfringens, Clostridium difficile, Clostridium novyi, Brucella abortus and/or Candidatus helicobacter suis. The use of antigens derived from one or several of Mycoplasma hyopneumoniae, Actinobacillus pleuropneumoniae, Actinobacillus suis, Haemophilus parasuis, Pasteurella multocida type A (toxins), Pasteurella multocida type D (toxins), Bordetella bronchiseptica, Isospora suis, Brachyspira hyodysenteriae, Brachyspira pilosicoli, Lawsonia intracellularis, Erysipelothrix rhusiopathiae, Eschericia coli and/or Salmonella enterica is specifically preferred.

The further viral or bacterial antigen may be present in the multivalent vaccine as an attenuated or inactivated virus or bacterium. Alternatively the multivalent vaccine may comprise a nucleic acid sequence encoding one or several of the further viral or bacterial antigens. That sequence may be within the same nucleic acid molecule that also encodes the TGEV and PRRSV sequences or it may be present in the vaccine as a seperate nucleic acid molecule.

The multivalent vaccine may further comprise nucleic acid sequences encoding proteins which will confer protection against pig pathogens. Respective nucleic acid sequences could for example code for one or several antibodies having specificity for bacterial or viral diseases. Alternatively or additionally, the nucleic acid may code for the sequence of the porcine prion protein and may thus be used to vaccinate against diseases caused by prions. Again, the sequences encoding proteins which will confer protection against pig pathogens may be within the same nucleic acid molecule that also encodes the TGEV and PRRSV sequences or may be present in the vaccine as a seperate nucleic acid molecule.

These vaccines may be administered in accordance with methods routinely used in the art. Specifically vaccine may be administered by intramuscular, intravenous, intranasal, intravaginal, oronasal administration or any other common method known in the art.

The vaccine preferably contains the TGEV/PRRSV nucleic acids in concentration producing a live viral titer in the range of about 10⁴ to 10⁹, most preferably about 10⁵ to 10⁸. The live viral titer is determined as plaque forming units (Pfu) in ST cells.

The vaccines of the present invention allow one of ordinary skill to diagnose whether an animal is infected with a wild-type virus or has been vaccinated. According to a further aspect, the present invention is thus directed to methods for diagnosing whether an animal is infected with a virus or has been vaccinated using a vaccine of the present invention, which methods comprise steps, wherein the diagnosis uses antibodies specific for proteins of the wildtype virus not expressed by the vaccine strain (i. e., 3a, 3b, 7 or E). Differentiation of TGEV vaccinated animals from TGEV infected animals could alternatively be carried out using RT-PCR and sequence markers introduced into the recombinant TGEV genome at positions 6752, 18997, 20460, and 21369, which should encode G, C, T, and C, respectively.

The differentiation between vaccinated animals and wildtype PRRSV infected animals can be carried out using antibodies specific for proteins not present in the recombinant virus.

### Brief description of the Figures:

Figure 1 shows the schematic structure of the cDNA encoding the pBAC-TGEV^{FL}-SPTV-TRS3a-ORF5-TRS22N-ORF6 (C306T). As shown the heterologous PRRSV genes ORF5 and ORF6 were cloned in the same rTGEV viral vector.
Figure 2 shows the expression of Gp5 (ORF5) of PRRSV by the viral clones which was evaluated by immunofluorescence.

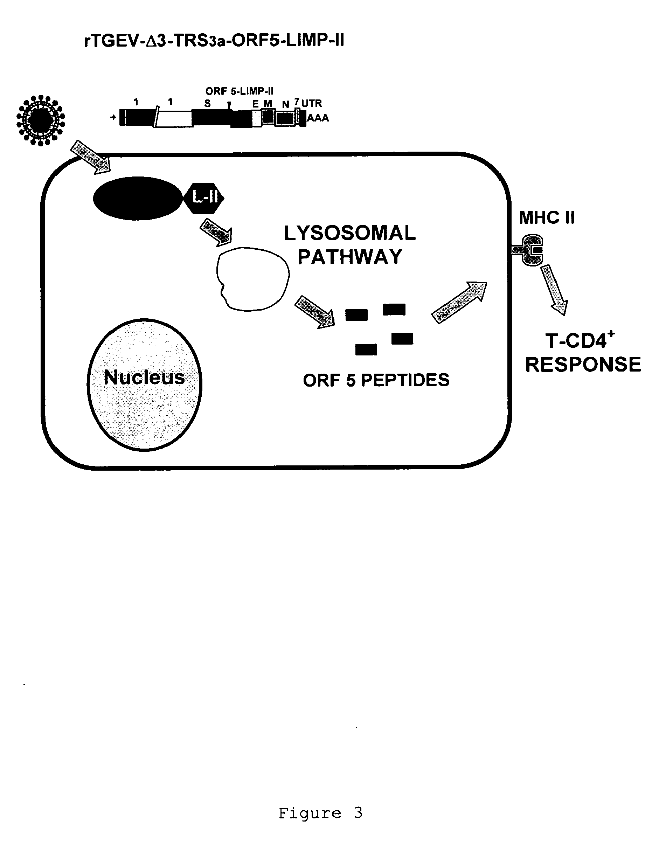

Figure 3 shows the schematic structure of the cDNA encoding a ORF5-LIMP-II fusion protein and its effect on antigen presentation by the infected cell.

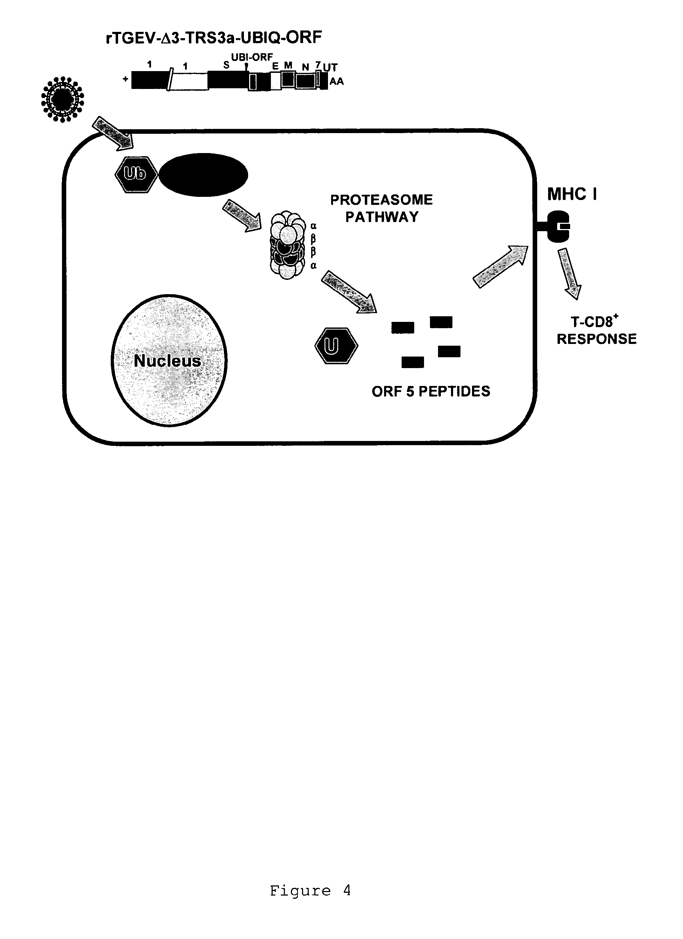

Figure 4 shows the schematic structure of the cDNA encoding a Ub-ORF5 fusion protein and its effect on antigen presentation by the infected cell.
Figure 5 shows the result from the in vivo experiment using rTGEVs expressing PRRSV Gp5. Weight increase is represented vs. days postinfection.
Figure 6 shows the result from the in vivo experiment using rTGEVs expressing Gp5. Protection was evaluated as the absence of antibodies against PRRSV N protein, that is an internal protein of the virus.
Figure 7 shows the scheme of the in vivo protocol followed to analyze the protection conferred by the rTGEV expressing Gp5 and Gp6.

The following examples illustrate the construction and use of the nucleic acids according to the invention.

### EXAMPLE 1

### Growth of Eukaryotic Cells

TGEV growth, titration, and purification were performed in ST (swine testicle) cells, a cell line obtained from epithelial cells of fetal pig testicles (McClurkin and Norman, 1966). ST cells were obtained from L. Kemeny (National Animal Disease Centre, Ames, Iowa, USA).

Plasmid transfections assays were performed in Baby Hamster Kidney cells (BHK-21) stably transformed with the gene coding for the porcine aminopeptidase N (BHK-pAPN) (Laude et al., 1990). ST cells were cultivated in DMEM (Dulbecco's Modified Eagle Medium) supplemented with 10% fetal calf serum (FCS) (GIBCO-BRL), 50 mg/mL gentamicine, 2 mM glutamine, and 1% non-essential amino acids.

The BHK-21 stably transformed with the gene encoding for the porcine aminopeptidase N (BHK-pAPN) were grown in DMEM (Dulbecco's Modified Eagle Medium) supplemented with 2% fetal calf serum (FCS) (GIBCO-BRL), 50 mg/mL gentamicine, 2 mM glutamine, and 1% non-essential amino acids and Geneticine (G418) (1,5 mg/ml) as a selection agent.

### EXAMPLE 2

### Transformation of bacteria by plasmid electroporation

### Bacterial strains:

*Escherichia coli* DH10B (Gibco/BRL) (Hanahan et al., 1991) was the host for all the plasmids constructed. The genotype of this bacterial strain is: F⁻mcr A Δ (mrr-hsdRMS-mcrBC) φ80d*lac*ZΔM15 *Δlac*X74 *deo*R *rec*A1 *end*A1 *ara*D139 (*ara,leu*) 7697 *gal*U *gal*K λ⁻ *rsp*L *nup*G.

### Preparation of competent bacteria:

For amplification and production of electroporation-competent *E. coli* DH10B bacteria, the bacteria were grown in a SOB medium. Were inoculated 10 mL of SOB medium (20 g/L tryptone, 5 g/L yeast extract, 0,5 g/L NaCl) with a colony from a fresh plate, and were incubated for 12 h at 37°C under agitation. With 2 mL of this culture, 1 L of SOB medium supplemented was inoculated, and the culture was grown at 37°C to an optical density of 600 nm, between 0.8 and 0.9 absorbance units. Then the culture was cooled on ice for 20 min, and the bacteria were centrifuged in the Sorvall GSA rotor at 4,000 G for 15 min at 4°C. The bacteria were resuspended cold in 1 L of 10% glycerol. The bacteria suspension was centrifuged again and resuspended in 500 mL of 10% cold glycerol. The bacteria were sedimented and resuspended in 250 mL of 10% cold glycerol. Finally, the bacteria were centrifuged and resuspended in 3 mL of 10% glycerol. The final suspension was divided into aliquots parts of 50 µL and 100 µL and were kept at -70°C until they were used for electroporation. The transformation efficiency of the bacteria was calculated by electroporation with a known concentration of a pBluescript plasmid as a reference, and was reproducibly at about 10⁹ colonies/µg of DNA.

### Transformation of bacteria by plasmid electroporation:

50 µL of transformation-competent bacteria were mixed with 1 µL of each reaction mixture, or 10 ng of purified plasmid to the bacteria and incubated on ice for 1 min. Then, the mixture was transferred to 0.2 cm electroporation trays (Bio-Rad), and were transformed by a 2.5 kV electric pulse, 25 µF and 200 Ω in a "Gene Pulser" electroporator (Bio-Rad). After adding 1 mL of cold LB medium, the bacteria were incubated at 37°C under agitation for 1 h. Between 50 and 100 µL of the suspension of transformed bacteria were seeded in Petri dishes with LB (Luria-Bertani medium) in a solid medium (15 g/L agar) supplemented with ampicillin (100 µg/mL) or chloramphenicol (34 µg/mL). The bacteria grew for 16 h at 37°C (Bullock, et al. 1987).

For production and purification of plasmids, the bacteria transformed with plasmids, that conferred ampicillin or chloramphenicol resistance, were grown from an isolated colony on a plate, in a liquid LB medium supplemented with 100 µg/mL of ampicillin or 34 µg/mL of chloramphenicol.

### EXAMPLE 3

### Plasmids for cloning of PCR products

The pGEM-T (Promega) plasmid was used to clone PCR products. This plasmid contains the T7 and SP6 bacteriophage promoters separated by the LacZ gene, interrupted by two protuberant T sequences between a multicloning sequences. This plasmid confers ampicillin resistance for its selection.

### EXAMPLE 4

### Manipulation of DNA

### Cloning and restriction enzymes:

For the manipulation and cloning of DNA, the restriction enzymes *Bam*HI, *Bbs* I, *Blp* I, *Eco* RI, *Mlu* I, *Swa* I, *Xcm* I, *Xho* I, were acquired from ROCHE or from New England Biolabs. Dephosphorylation of the DNA terminals, was done with shrimp alkaline phosphatase (SAP) (USB). A DNA ligation as T4 phage DNA ligase (New England Biolabs) was used. All the treatment with restriction enzymes, dephosphorylation, and DNA ligation were carried out by standard protocols previously described (Sambrook et al., 1989).

### Polymerase chain reaction (PCR):

To amplify DNA from a matrix, frequently plasmids, 50-100 ng of DNA plasmid was mixed with the corresponding oligonucleotides (10 µM), 0.25 mM deoxynucleotides triphosphate (ATP, GTP, TTP, and CTP), 1.25 mM MgCl₂, PCR buffer (10 mM Tris-HCl, pH 8.3, 50 mM KCl) and 2.5 U of Taq Gold DNA polymerase (*Thermus aquaticus)* (Roche), in a final volume of 50 µL. The reactions were carried out in the GeneAmp PCR System 9600 thermocycler from Perkin Elmer.

### Separation of DNA by agarose gel electrophoresis:

To separate DNA fragments, 1% agarose gels were used with ethydium bromide (1 µg/mL) in a 1X TAE buffer (40 mM Tris-acetate, 1mM EDTA).

### Purification of DNA:

The bacterial plasmids grown in the presence of the selection antibiotics were purified using the "Qiaprep Spin Miniprep kit" (Qiagen) to prepare small quantities of plasmid DNA, and the "Qiafilter Midi-Plasmid Kit" system (Qiagen) to prepare intermediate quantities of plasmid DNA. The DNA obtained from agarose gels was purified using the "QiaEx II Gel Extraction Kit" system (Qiagen). Purification of the PCR products was carried out by means of the system "QIA quick PCR Purification Kit" (Qiagen). In all cases, the manufacturer's instructions were followed.

### EXAMPLE 5

### RNA analysis

For analysis of the RNA produced in infections with TGEV clone PUR46-MAD, confluent monolayers of ST cells grown in 60 mm diameter culture plates (NUNC) were infected with viral inocula at a MOI of 1. The cells were lysed at 16 hpi [hours post infection] using a "RNeasy Mini Kit" (Qiagen) , following the protocol provided by the commercial firm (Qiagen). The RNA was purified and resuspended in 40 µL of water treated with DEPC [diethyl polycarbonate] and 20 U of RNAse inhibitor (Roche).

### EXAMPLE 6

### Transfection and Recovery of infectious TGEV from cDNAs Clones

BHK-pAPN cells (Delmas, 1994) were grown in Dulbecco's modified Eagle's medium (DMEM) supplemented with 2% fetal calf serum and containing Geneticin (G418) (1,5 mg/ml) as a selection agent. BHK-pAPN cells were grown to 60% confluence in 35-mm-diameter plates and transfected with 10µg of pBAC-TGEV^{FL}-SPTV-TRS3a-ORF5-TRS22N-ORF6 (C306T) plasmid with 15µl of lipofectin (GIBCO Life Technologies) according to the manufacturer's specifications. The cells were incubated at 37°C 5% CO₂ and after 6 h the transfection medium was replaced with fresh DMEM containing 5% (vol/vol) FBS. Two days later (referred to as passage 0), the cells supernatants were harvested and passaged four times on fresh ST monolayer to increase rTGEV titer. Virus titers were determined by plaque titration.

Alternatively, ST cells were grown in 25 cm² culture flask using DMEM (Dubelco's Minimal Essential Medium) 10% SFB (Serum Fetal Bovine) at 90% of confluence and infected at a MOI (multiplicity of infection) of 1 plaque unit formation per cell. The supernatant was recovered after 48 hours and titrated by plaque limit dilution.

### Plaque titration:

Titration of viral stocks was made by plaque limit dilution assay on 24-well cultured ST cells to quantify the number of infective particles. ST cells were grown in 24-multiwell culture plate at 90 % of confluence. Recombinant TGEV viruses were 10-fold serially diluted (10E-1, 10E-2, 10E-3, 10E-4, 10E-5, 10E-6,etc). The different virus dilutions were added to each well of the 24-well plate and incubated for 1 hour at 37°C, 5%CO₂. After that hour the supernatant containing the virus was removed from the ST monolayer and quickly an overlay AGAR was added onto the monolayer. The overlay AGAR was prepared using 1 part of 2X DMEM (Dubelco's Minimal Essential Medium) and 1 part of 1% purified AGAR in ddH₂O. After overlaying the cells the multi-well plate was kept for 15 minutes at room temperature to solidify the agarose and then was placed in a controlled incubator for 48 hours at 37°C, 5%CO₂.

In order to count the viral plaques the infected ST cells monolayer were fixed with 10% formol and stained with crystal violet 0.1% for 30 minutes. The well was washed with destilated water and dryed at room temperature to finally count the plaques to find the virus titer.

TGEV and PRRSV protein expression were analysed by standard immunofluorescence techniques.

### EXAMPLE 7

### Generation of rTGEV

The porcine transmissible gastroenteritis virus (TGEV) used belongs to the group of Purdue isolates, and was obtained in Indiana in 1946 (Doyle and Hutchings, 1946). The virus was adapted to grow in cell cultures (Haelterman and Pensaert, 1967), and was provided by E. H. Bohl (Ohio State University, Wooster Ohio). This TGEV isolate has been passaged in ST cells 115 times, and has been cloned five times consecutively in Dr. Luis Enjuanes laboratory (Centro Nacional de Biotecnologia, Madrid, Spain). The clone selected was labelled PUR46-CC120-MAD, abbreviated PUR46-MAD. This is an attenuated virus that grows well in cell cultures, and reaches titers between 10⁸ and 10⁹ PFU/mL.

rTGEV viruses were generated from pBAC-TGEV constructs containing the S gene from the virulent TGEV strain PUR-C11 (S_{C11}) as described (Alamazan et al., 2000 ; Gonzalez et al., 2002). Viruses containing the S gene (encoding the TGEV spike protein) from the attenuated strain PTV (S_{PTV}) were derived from the corresponding pBAC-TGEV vectors with S_{C11} by replacing this gene by the S_{PTV} of the respiratory strain.

### EXAMPLE 8

### Construction of a recombinant TGEV vector expressing Gp5 and M protein of PRRSV

In order to increase the cloning capacity of the TGEV single genome, the non-essential genes ORFs 3a and 3b were eliminated from the full-length cDNA clone, creating a deletion in the TGEV genome. The heterologous genes ORF5 and ORF6 encoding PRRSV proteins Gp5 and M were inserted in the cDNA construct, replacing the deleted TGEV ORFs 3a and 3b.

ORF5 (606nt) (SEQ ID NO: 2), was cloned in cDNA constructs replacing the TGEV genes 3a and 3b. The gene was amplified by PCR using oligonucleotides PpuMI-ORF5 VS (5'-AACAGGTCCTACCATGAGATGTTCTCACAAATTGGGG-3') (SEQ ID NO:4) and Blp-ORF5 RS mut (5'-CCGCTAAGCCTAGGCTTCCCATTGCTCAGCCGAAGTCC-3') (SEQ ID NO:5). PCR product was digested with *Ppu*MI and *Blp*I and cloned in the same sites of plasmid pSL-TGEV-AvrII, comprising nt 22965 to 25865 from the TGEV genome including the 3a and 3b deletion, generating plasmid pSL-AvrII-Δ3-PpuMI-ORF5. To obtain the infectious cDNAs this plasmid was digested with *Avr*II and the insert containing ORFs 5 was cloned in the same sites of pBAC-Sc11 or pBAC-S_{PTV}(PacI/MluI), to obtain vectors with enteric and respiratory tropism, respectively. The sequences of pBAC plasmids and of the infectious TGEV cDNA clone have been previously reported (PCT/EP00/12063).

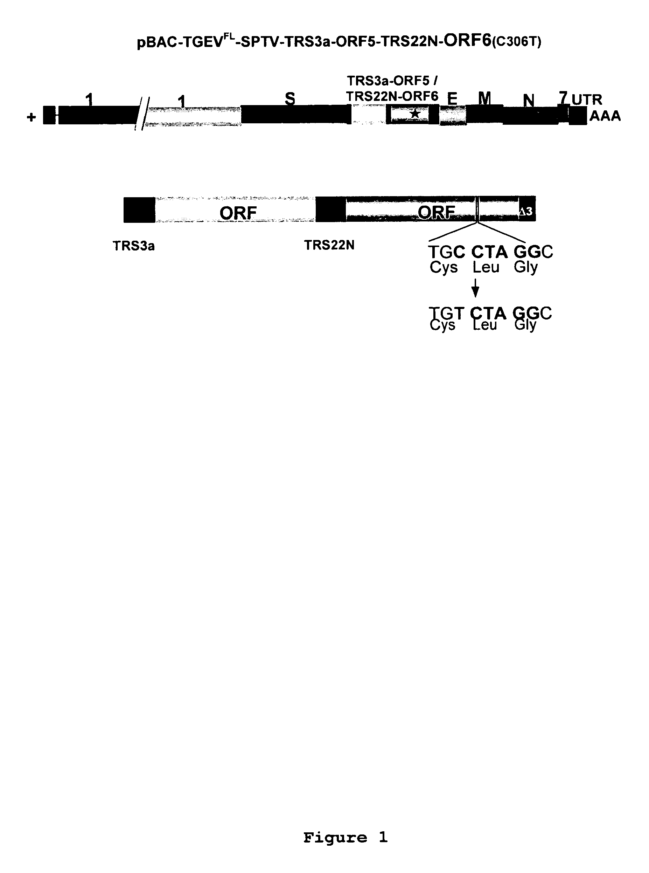

ORF6 from PRRSV Olot91 strain (SEQ ID NO:3) was amplified by overlapping PCR to introduce a silent mutation eliminating an *Avr*II restriction site, due to cloning restrictions, taken into account the *Sus scrofa* codon usage. In a first PCR, oligonucleotides BlpIORF6 VS (5'-CGGCTGAGCAATGGGAAGCCTAGAAAATTATTACATATGGTATAACTAAACAAAATGG-GAAGCCTAGACGATTTTTG-3') (SEQ ID NO:6), underlined sequence being the TRS22N, and ORF6-C306T-RS (5'-GCCGGCCTA GACAACACAATC-3') (SEQ ID NO:7) were used. Using a similar procedure, oligonucleotides ORF6-C306T-VS (5'-GATTGTGTTGTCTAGGCCGGC-3') (SEQ ID NO:8) and BlpIORF6rs new (5'-GCTA AGCTTACCGGCCATACTT GACGAGG-3') (SEQ ID NO:9) were used. Using these PCR products and oligonucleotides BlpIORF6 VS and BlpIORF6rs new, the final product (574 nt) was obtained. This PCR product was digested with *Blp*I and cloned in the same site of pSL-AvrII-Δ3-PpuMI-ORF5, generating plasmid pSL-TRS3a-ORF5-TRS22N-ORF6(C306T). To obtain the infectious cDNA pBAC-SPTV-TRS3a-ORF5-TRS22N-ORF6(C306T), plasmid pSL-TRS3a-ORF5-TRS22N-ORF6(C306T) was digested with *Avr*II and the insert containing ORFs 5 and 6 was cloned in the same sites of pBAC-SPTV(PacI/MluI) (Fig. 1). The same cloning procedure was used to obtain the cDNA encoding the TGEV with enteric tropism (adapted to grow in cell culture), expressing PRRSV Gp5 and M.

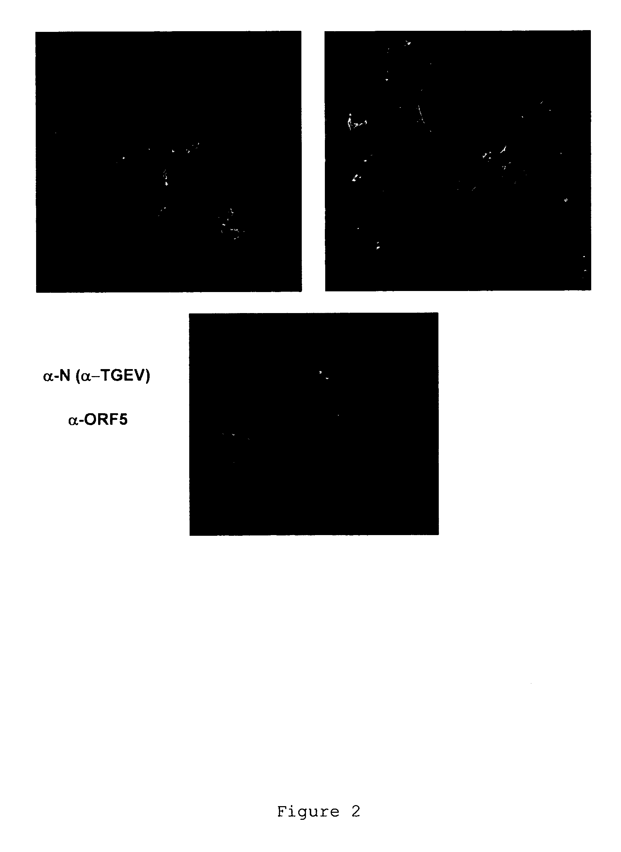

The recombinant virus with respiratory tropism, rTGEV-SPTV-TRS3a-ORF5-TRS22N-ORF6(C306T), was rescued and plaque cloned three times. The presence of the mRNAs for ORF5 and ORF6 was checked by RT-PCR. One expressing clone was selected and, after two passages in cell culture, mRNA-5 and mRNA-6 were detected, indicating that the virus was stable. Nevertheless, to improve expression levels by adapting the recombinant virus to grow in ST cells, two more plaque cloning steps were performed. Expression of Gp5 by the viral clones was evaluated by immunofluorescence and the amount of infected cells expressing Gp5 was quantified. The selected virus (rTGEV-SPTV-TRS3a-ORF5-TRS22N-ORF6(C306T)) expressed high amounts of Gp5 in the 72.6 % of the infected cells evaluated by immunofluorescence (Fig.2) and fluorescence-activated cell sorting (FACS).

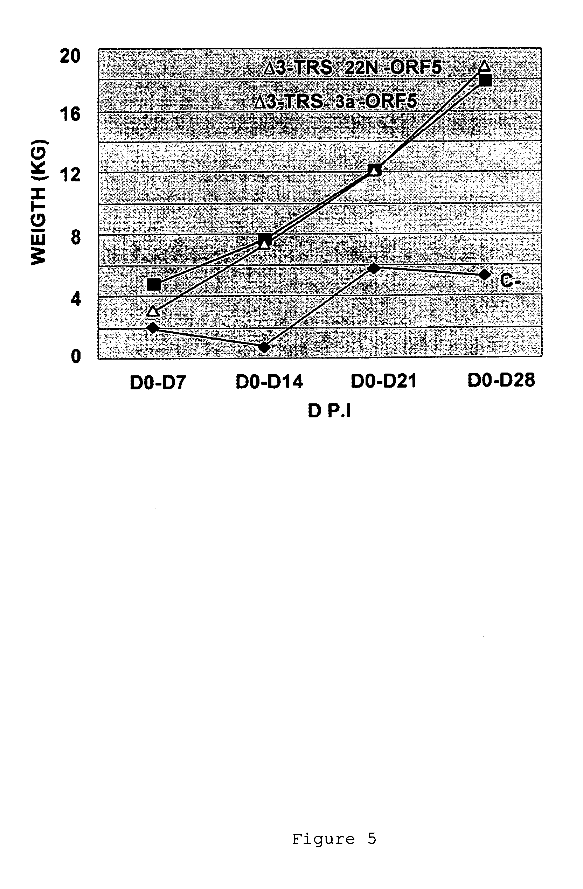

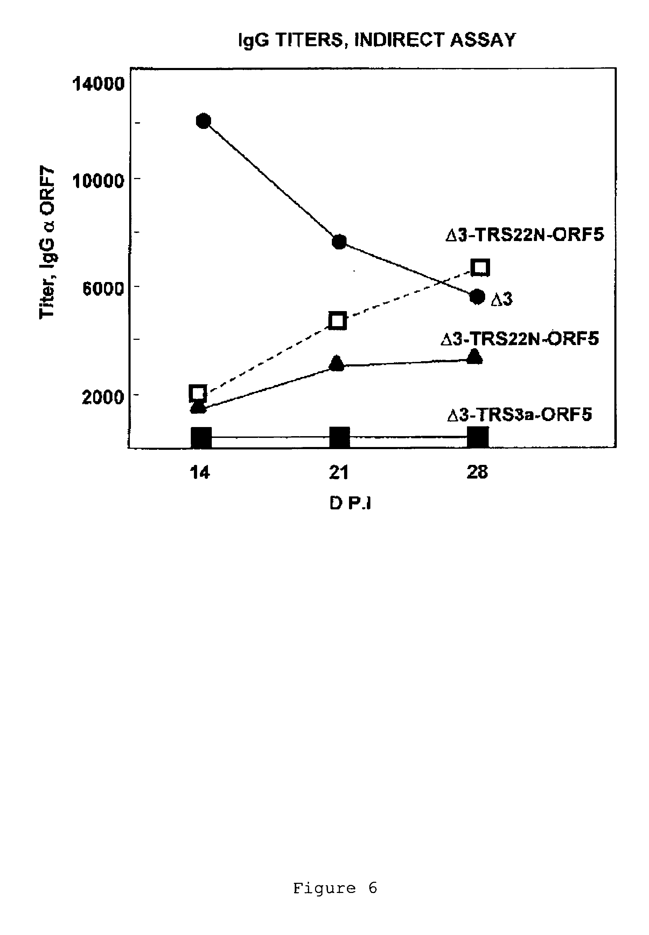

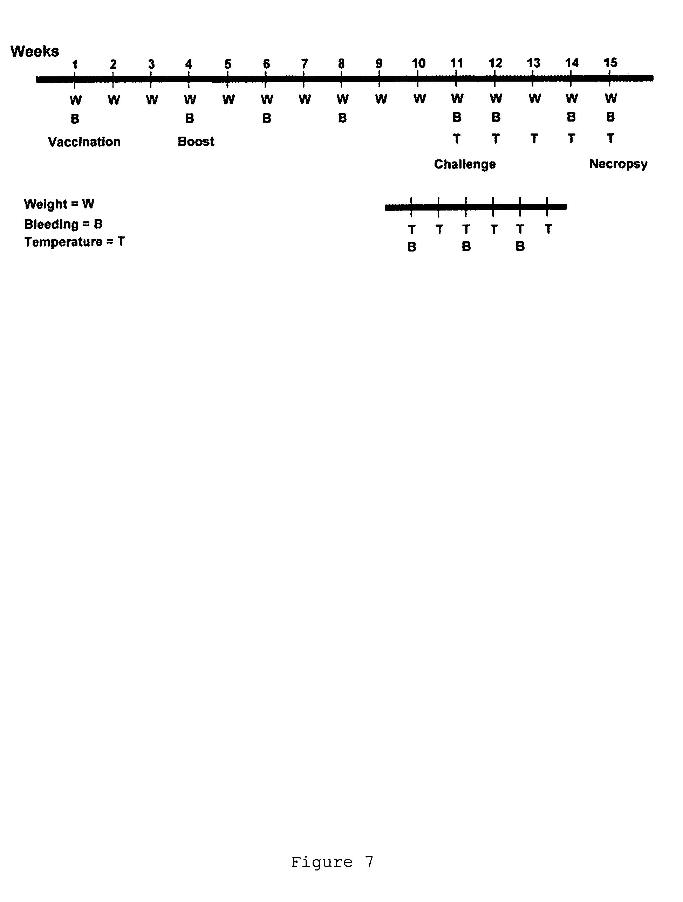

*In vivo* experiments using the selected recombinant virus showed high antibodies titers against PRRSV, including neutralizing antibodies, and also protection in vaccinated pigs after challenge. Experiments vaccinating pigs with the rTGEV expressing Gp5, demonstrated a certain degree of protection shown as a increment of the weight of the vaccinated animals (Fig. 5). This protection was also demonstrated by the reduction in the level of antibodies against PRRSV N protein in the serum of the vaccinated animals after challenge (Fig. 6). The immunization protocol for the analysis of the rTGEV expressing Gp5 and M proteins is shown in Fig. 7. Pigs were inoculated intranasally and intragastrically with rTGEV-expressing ORFs 5 and 6 (1x10⁸ Pfu/animal). The control of virus inoculum indicated that in tissue cultures (ST cells) 100% of the cells infected with the vector expressed ORF6 protein and more than 80% of these cells expressed ORF5 protein.

The serums from week 8 were analyzed by ELISA and antibodies against TGEV and PRRSV were found in the animals inoculated with the rTGEV expressing Gp5 and Gp6. In a neutralization assay neutralizing antibodies against PRRSV were found.

### Example 9

### Vector expressing ORFS-LIMP-II fusion protein

The recombinant vector was engineered using the same cloning protocol as described above. The expression of the fusion protein was directed by TRS-3a, and the vector was designed with enteric tropism. The vector pBAC-SC11-TRS3a-ORF5-LIMPII was obtained by cloning the PRRSV ORF5 fused to the last 20 aa (SEQ ID NO:11) of porcine LIMP-II (GeneBank accession number AAS55916), containing the lysosomal targeting sequence of this protein.

Stable recombinant virus was recovered from pBAC-Sc11-TRS3a-ORF5-LIMPII cDNA.

Gp5-LIMPII expression was detected by Western-blot and immunofluorescence using specific inhibitors of lysosomal pH (i.e., chloroquine, NH₄Cl). High and stable expression of the antigen was found.

This recombinant virus was used to perform *in vivo* experiments as described in example 8 and shown in Figure 7. High neutralizing antibodies titers and protection against PRRSV challenge were found.

### Bibliography:

Alamazan, F., Gonzales J.M., Penzes Z., Izeta A., Calvo E., Plana-Duran J., and Luis Enjuanes (2000). Proc. Natl. Acad. Sci. USA 97: 5516-5521.

Botner, A., Strandbygaard, B., Sorensen, K. J., Have, P., Madsen, K. G., Madsen, E. S. & Alexandersen, S. (1997). Appearance of acute PRRS-like symptoms in sow herds after vaccination with a modified live PRRS vaccine. Vet. Rec. 141, 497-499.

Bullock, W., Fernández, J.M. and Short, J.M. (1987). XL1-Blue: a high efficiency plasmid transforming recA E.coli strain with P-galactosidadse selection. Biotechniques 8:26-27.

Chang, K.-Y., and Tinoco, I. (1994). Characterization of a "kissing" hairpin complex derived from the human immunodeficiency virus genome. Proc. Natl. Acad. Sci. USA 91, 8705-8709.

Delmas B, Gelfi J., Kut E., Sjostrom H., Noren O., Laude H. (1994). Determinants essential for the transmissible gastroenteritis virus-receptor interaction reside within a domain of aminopeptidase-N that is distinct from the enzymatic site. J Virol. 68(8): 5216-24.

Doyle, L.P. and Hutchings, L.M. (1946). A transmissible gastroenteritis in pigs. J. Amer. Vet. Med. Assoc. 108:257-259.

Enjuanes L., et al. (2003). Virus based vectors for gene expression in mammalian cells; Coronaviruses; in Gene transfer and expression in mammalian cells; Ed. S.C. Makrides, p. 151-158.

Gonzalez, J.M., Penzes Z., Almazan F., Calvo E., and Luis Enjuanes (2002). Stabilization of a full-length infectious cDNA clone of transmissible gastroenteritis coronavirus by the insertion of an intron. J. Virol. 76: 4655-4661.

Haelterman E.O. and Pensaert M.B. (1967). Pathogenesis of transmissible gastroenteritis of swine. Proc. 18th World Vet. Congress 2:569-572.

Hanahan D., Jessee J., Bloom F.R. (1991). Plasmid transformation of Escherichia coli and other bacteria. Methods Enzymol. 204: 63-113.

Izeta, A., Smerdou, C., Alonso, S., Penzes, Z., Méndez, A., Plana-Durán, J., and Enjuanes, L. (1999). Replication and packaging of transmissible gastroenteritis coronavirus-derived synthetic minigenomes. J. Virol.73, 1535-1545.

Kiyono et al. (1996). Mucosal Vaccines. Academic Press, New York.

Laude, H., Rasschaert D., Delmas B., Godet M., Gelfi J., and Bernard C. (1990). Molecular biology of transmissible gastroenteritis virus. Vet. Microbiol. 23: 147-154.

Lopez, O. J. & Osorio, F. A. (2004). Role of neutralizing antibodies in PRRSV protective immunity. Vet Immunol Immunopathol 102, 155-163.

McClurkin, A.W. and Noman, J.O. (1966). Studies on transmissible gastroenteritis of swine. II. Selected characteristics of a cytopathogenic virus common to five isolates from transmissible gastroenteritis. Can. J. Comp. Med. Vet. Sci. 30:190-198.

Meng, X. J. (2000). Heterogeneity of porcine reproductive and respiratory syndrome virus: implications for current vaccine efficacy and future vacine development. Vet. Microbiol. 74, 309-329.

Murtaugh, M. P., Xiao, Z. & Zuckermann, F. (1995). Comparison of the structural protein coding sequences of the VR-2332 and Lelystad virus strains of the PRRS virus. Arch. Virol.140, 1451-1460.

Nielsen, H. S., Oleksiewicz, M. B., Forsberg, R., Stadejek, T., Botner, A. & Storgaard, T. (2001). Reversion of a live porcine reproductive and respiratory syndrome virus vaccine investigated by parallel mutations. J. Gen. Virol. 82, 1263-1272.

Ortego J., Escors D., Laude H., and Luis Enjuanes (2002). Generation of a replication-competent, propagation deficient virus vector based on the transmissible gastroenteritis coronavirus genome. J.Virol. 76:11518-11529.

Ortego J., Sola I., Almazan F., Ceriani J.E., Riquelme C., Balach M., Plana-Duran J. and Luis Enjuanes (2003). Transmissible gastroenteritis coronavirus gene 7 is not essential but influences in vivo replication and virulence. Virology 308: 13-22.

Osorio, F. A., Zuckermann, F., Wills, R., Meier, W., Christian, S., Galeota, J. & Doster, A. R. (1998). PRRSV: comparison of commercial vaccines in their ability to induce protection against current PRRSV strains of high virulence. In Allen D. Lennan Swine Conf., pp. 179-182.

Penzes Z., Gonzales J.M., Calvo E., Izeta A., Smerdou C., Mendez A., Sanches C.M., Sola I., Almazan F., Enjuanes L. (2001). Complete genome sequence of Transmissible Gastroenteritis Coronavirus PUR46-MAD clone and evolution of the purdue virus cluster. Virus Genes 23:1, 105-118.

Sambrook et al. (1989). Molecular Cloning: A Laboratory Manual. Ed Cold Spring Harbor Laboratory.

Sánchez, C. M., Izeta, A., Sánchez-Morgado, J. M., Alonso, S., Sola, I., Balasch, M., Plana-Durán, J. and Enjuanes, L. (1999). Targeted recombination demonstrates that the spike gene of transmissible gastroenteritis coronavirus is a determinant of its enteric tropism and virulence. J. Virol. 73:7607-7618.

Sawicki & Sawicki (1990). Coronavirus transcription: subgenomic mouse hepatitis virus replicative intermediates function in RNA synthesis. J Virol. 64(3):1050-6.

Sethna, P. B., Hung, S.-L., and Brian, D. A. (1989). Coronavirus subgenomic minus-strand RNAs and the potential for mRNA replicons. Proc. Natl. Acad. Sci. USA 86, 5626-5630.

Siddell, S. G. (1995). "The Coronaviridae." The Viruses (H. Fraenkel-Conrat, and R. R. Wagner, Eds.) Plenum Press, New York.

Sola I, Alonso S., Zúñiga S., Balasch M., Plana-Duran J., Enjuanes L. (2003). Engineering the transmissible gastroenteritis virus genome as an expression vector inducing lactogenic immunity. J. Virol. 77:4357-4369.

Toka, F.N. et al. (2004). Molecular adjuvants for mucosal immunity. Immunol Rev. 199:100-112.

van der Most, R. G., and Spaan, W. J. M. (1995). Coronavirus replication, transcription, and RNA recombination. In "The Coronaviridae" (S. G. Siddell, Ed.), pp. 11-31. Plenum Press, New York.

Wensvoort, G., Terpstra, C., Pol, J. M., ter Laak, E. A., Bloemraad, M., de Kluyver, E. P., Kragten, C., van Buiten, L., den Besten, A., Wagenaar, F. & et al. (1991). Mystery swine disease in The Netherlands: the isolation of Lelystad virus. Vet. Q. 13, 121-130.

## Claims

1. Nucleic acid comprising:
(a) sequences of a replication competent transmissible gastroenteritis virus (TGEV), which sequences encode a TGEV replicase under the control of expression regulatory sequences, wherein the replicase is expressed in a host cell and will initiate replication of the nucleic acid and thus increase the number of nucleic acids in the cell; and
(d) a sequence encoding at least one neutralizing epitope of ORF5 of porcine reproductive and respiratory syndrome virus (PRRSV), which sequence includes a disulfide bridge forming residue; and
(e) a sequence encoding at least one further polypeptide capable of increasing an immune response against PRRSV.

2. Nucleic acid comprising:
(a) sequences of a replication competent transmissible gastroenteritis virus (TGEV), which sequences encode a TGEV replicase under the control of expression regulatory sequences so that expression of the replicase in a cell containing the nucleic acid will initiate replication of the nucleic acid and thus increase the number of nucleic acids in the cell; and
(b) sequences encoding multimers of one or several neutralizing epitopes of ORF5 of PRRSV and at least one further polypeptide capable of increasing an immune response against PRRSV.

3. Nucleic acid according to claim 1 or 2, wherein the sequence encoding said neutralizing epitope of ORF 5 has been modified to knock out glycosylation sites that interfere with induction of antibodies.

4. Nucleic acid according to any of claims 1 to 3, wherein the nucleic acid encodes a TGEV replicase and a sequence encoding the TGEV N protein.

5. Nucleic acid according to any of claims 1 to 4, wherein the replication competent TGEV vector is not infectious.

6. Nucleic acid according to any of claims 1 to 4, wherein the replication competent TGEV vector is infectious.

7. Nucleic acid according to claim 6, wherein the nucleic acid further comprises one or more of the following TGEV genes: S, E, M and/or N or sequences having a similarity of at least 60% to the given sequence.

8. Nucleic acid according to claim 6 or 7, wherein the TGEV infectious viral particles obtainable from the association of TGEV proteins and the nucleic acid sequences are attenuated viral particles.

9. Nucleic acid according to any of claims 6 to 8, wherein the S gene is derived from a respiratory virus.

10. Nucleic acid according to any of claims 6 to 8, wherein the S gene is derived from an enteric strain of TGEV.

11. Nucleic acid according to claim 10, wherein the S gene has been modified and has the SEQ ID NO: 1.

12. Nucleic acid according to any one of claims 1 to 11, wherein the sequence comprises a neutralizing epitope of ORF5 and a neutralizing epitope of ORF 6 of PRRSV.

13. Nucleic acid according to claim 12 wherein the sequence consists of a neutralizing epitope of ORF5 and a neutralizing epitope of ORF 6 of PRRSV.

14. Nucleic acid according to any one of claims 1 to 12, wherein the nucleic acid further encodes the lysosomal targeting signal of lysososmal integral membrane protein-II (LIMPII).

15. Nucleic acid according to claim 14, wherein said nucleic acid encodes a fusion protein consisting of LIMPII and ORF5 and/or ORF6 of PRRSV.

16. Nucleic acid comprising:
(a) sequences of a replication competent transmissible gastroenteritis virus (TGEV), which sequences encode a TGEV replicase under the control of expression regulatory sequences, wherein the replicase is expressed in a host cell and will initiate replication of the nucleic acid and thus increase the number of nucleic acids in the cell;
(b) residues 118 to 138 of SEQ ID NO:2 or a sequence having a similarity of 90% to these residues under the control of an expression regulatory sequence; and
(c) SEQ ID NO:3 or a sequence having a similarity of 90% to SEQ ID NO:3 under the control of an expression regulatory sequence; wherein
the sequences of (a) and (b) and (c) are each under the control of a different expression regulatory sequence.

17. Nucleic acid according to claim 16 further comprising the nucleic acid sequence SEQ ID NO:12, which encodes the lysosomal targeting signal of lysososmal integral membrane protein-II (LIMP-II) or a sequence having a similarity of 90% to SEQ ID NO:12.

18. Nucleic acid according to any one of claims 1 to 12 and 14 to 17, wherein the nucleic acid further encodes the p35 subunit of IL-12 or other interleukins.

19. Recombinant RNA encoded by a nucleic acid according to any of claims 1 to 18.

20. Vector comprising a nucleic acid according to one of claims 1 to 19.

21. Vector according to claim 20, wherein the vector is a cDNA vector.

22. Vector according to claim 21, wherein the vector is a BAC-TGEV^{FL} vector.

23. Vector according to any of claims 20 to 22, wherein the vector is capable of replicating the nucleic acid within a host cell.

24. Host cell comprising a vector according to one of claims 20 to 22.

25. Host cell according to claim 24, wherein the cell is a bacterial cell, a yeast cell, an insect cell, an animal cell or a human cell.

26. Host cell according to claim 25, wherein the cell is a porcine swine testis cell line, such as the cell line deposited under ATCC CRL-1746.

27. Virus particle comprising a nucleic acid according to any of claims 1 to 18 and at least one TGEV coat protein.

28. Virus particle according to claim 27, comprising all TGEV coat proteins of the native TGEV virus particle.

29. Polypeptide encoded by SEQ ID NO:13 or encoded by a sequence having a similarity of 90% to SEQ ID NO:13.

30. Polypeptide encoded by SEQ ID NO:14 or encoded by a sequence having a similarity of 90% to SEQ ID NO:14.

31. Pharmaceutical preparation comprising a nucleic acid according to one of claims 1 to 18, a viral RNA or vector according to one of claims 19 to 23 or a host cell according to one of claims 24 to 26 or a virus particle according to claim 27 or 28 or a polypeptide according to claim 29 or 30.

32. Pharmaceutical preparation according to claim 31 further comprising a pharmaceutically acceptable carrier, excipient and/or adjuvants.

33. Vaccine capable of protecting an animal against PRRSV comprising a nucleic acid according to one of claims 1 to 18, a viral RNA or vector according to one of claim 19 to 23, a host cell according to one of claims 24 to 26 or a virus particle according to claim 27 or 28 or a polypeptide according to claim 29 or 30.

34. Vaccine according to claim 33, wherein the vaccine is a multivalent vaccine capable to provide protection against one or several pig pathogens other than PRRSV.

35. Vaccine according to claim 34, further comprising antigens derived from other viral and/or bacterial pathogens and/or proteins which will provide immunity against pathogens.

36. Vaccine according to claim 34 or 35, further comprising one or several antigens derived from other viral pathogens selected from Swine Influenza Viruses, Porcine Parvovirus, Porcine Circovirus Type 2, Classical Swine Fever, African Swine Fever, Foot-and-Mouth Disease, Pseudo-Rabies Virus, Porcine Circovirus Type 1, Porcine Adenoviruses, Porcine Enteroviruses, Porcine Respiratory Coronavirus, Porcine Rotavirus, Encephalomyocarditis Virus, Porcine Epidemic Diarrhea Virus, Blue Eye Disease Viruses, Hepatitis E Virus and/or West Nile Virus, Nipah virus.

37. Vaccine according to any one of claims 34 to 36, further comprising one or several antigens derived from other viral pathogens selected from Swine Influenza Viruses, Porcine Parvovirus, Porcine Circovirus Type 2, Classical Swine Fever, African Swine Fever and/or Foot-and-Mouth Disease.

38. Vaccine according to any one of claims 34 to 36, further comprising one or several antigens derived from bacterial pathogens selected from Mycoplasma hyopneumoniae, Actinobacillus pleuropneumoniae, Actinobacillus suis, Haemophilus parasuis, Pasteurella multocida type A (toxins), Pasteurella multocida type D (toxins), Bordetella bronchiseptica, Isospora suis, Brachyspira hyodysenteriae, Brachyspira pilosicoli, Lawsonia intracellularis, Erysipelothrix rhusiopathiae, Eschericia coli, Salmonella enterica, Mycoplasma hyorinis, Streptococcus suis, Clostridium perfringens, Clostridium difficile, Clostridium novyi, Brucella abortus and/or Candidatus helicobacter suis.

39. Vaccine according to any one of claims 34 to 36, further comprising one or several antigens derived from bacterial pathogens selected from Mycoplasma hyopneumoniae, Actinobacillus pleuropneumoniae, Actinobacillus suis, Haemophilus parasuis, Pasteurella multocida type A (toxins), Pasteurella multocida type D (toxins), Bordetella bronchiseptica, Isospora suis, Brachyspira hyodysenteriae, Brachyspira pilosicoli, Lawsonia intracellularis, Erysipelothrix rhusiopathiae, Eschericia coli and/or Salmonella enterica.

40. Vaccine according to any one of claims 34 to 39, wherein the further viral or bacterial antigen is present in the multivalent vaccine as an attenuated or inactivated virus or bacterium or as a nucleic acid sequence encoding one or several of the further viral or bacterial antigens.

41. Vaccine according to any one of claims 34 to 40, further comprising nucleic acid sequences encoding proteins which will confer protection against pig pathogens, such as nucleic acid sequences encoding one or several antibodies having specificity for bacterial or viral diseases or nucleic acid sequences encoding the porcine prion protein.

42. Vaccine according to one of claims 33 to 41 further comprising a pharmaceutically acceptable carrier, excipient and/or adjuvants.

43. Vaccine according to one of claims 33 to 42, wherein the vaccine is suitable for vaccinating a swine, preferably a sow.

44. Vaccine according to one of claims 33 to 43, wherein the vaccine is capable of inducing both a systemic immune response and a mucosal immune response against infectious viral agents.


SEQUENCE LISTING

<110> Consejo Superior de Investigaciones Cientificas

<120> Nucleic Acid Sequences encoding Vaccines against Porcine Repro-
ductive and Respiratory Syndrome Virus (PRRSV)

<130> P69718

<160> 15

<170> PatentIn version 3.1

<210> 1
<211> 4350
<212> DNA
<213> PRRSV

<400> 1
atgaaaaaac tatttgtggt tttggtcgta atgccattga tttatggaga caattttcct 60

tgttctaaat tgactaatag aactataggc aaccagtgga atctcattga aaccttcctt 120

ctaaactata gtagtaggtt accacctaat tcagatgtgg tgttaggtga ttattttcct 180

actgtacaac cttggtttaa ttgcattcgc aataatagta atgaccttta tgttacactg 240

gaaaatctta aagcattgta ttgggattat gctacagaaa atatcacttg gaatcacaga 300

caacggttaa acgtagtcgt taatggatac ccatactcca tcacagttac aacaacccgc 360

aattttaatt ctgctgaagg tgctattata tgcatttgta agggctcacc acctactacc 420

accacagaat ctagtttgac ttgcaattgg ggtagtgagt gcaggttaaa ccataagttc 480

cctatatgtc cttctaattc agaggcaaat tgtggtaata tgctgtatgg cctacaatgg 540

tttgcagatg aggttgttgc ttatttacat ggtgctagtt accgtattag ttttgaaaat 600

caatggtctg gcactgtcac atttggtgat atgcgtgcga caacattaga agtcgctggc 660

acgcttgtag acctttggtg gtttaatcct gtttatgatg tcagttatta tagggttaat 720

aataaaaatg gtactaccgt agtttccaat tgcactgatc aatgtgctag ttatgtggct 780

aatgttttta ctacacagcc aggaggtttt ataccatcag attttagttt taataattgg 840

ttccttctaa ctaatagctc cacgttggtt agtggtaaat tagttaccaa acagccgtta 900

ttagttaatt gcttatggcc agtccctagc tttgaagaag cagcttctac attttgtttt 960

gagggtgctg gctttgatca atgtaatggt gctgttttaa ataatactgt agacgtcatt 1020

aggttcaacc ttaattttac tacaaatgta caatcaggta agggtgccac agtgttttca 1080

ttgaacacaa cgggtggtgt cactcttgaa atttcatgtt ataatgatac agtgagtgac 1140

tcgagctttt tcagttacgg tgaaattccg ttcggcgtaa ctgatggacc acggtactgt 1200

tacgtacact ataatggcac agctcttaag tatttaggaa cattaccacc tagtgtcaag 1260

gagattgcta ttagtaagtg gggccatttt tatattaatg gttacaattt ctttagcaca 1320

tttcctattg attgtatatc ttttaatttg accactggtg atagtgacgt tttctggaca 1380

atagcttaca catcgtacac tgaagcatta gtacaagttg aaaacacagc tattacaaag 1440

gtgacgtatt gtaatagtca cgttaataac attaaatgct ctcaaattac tgctaatttg 1500

aataatggat tttatcctgt ttcttcaagt gaagttggtc ttgtcaataa gagtgttgtg 1560

ttactaccta gcttttacac acataccatt gttaacataa ctattggtct tggtatgaag 1620

cgtagtggtt atggtcaacc catagcctca acattaagta acatcacact accaatgcag 1680

gatcacaaca ccgatgtgta ctgtattcgt tctgaccaat tttcagttta tgttcattct 1740

acttgcaaaa gtgctttatg ggacaatatt tttaagcgaa actgcacgga cgttttagat 1800

gccacagctg ttataaaaac tggtacttgt cctttctcat ttgataaatt gaacaattac 1860

ttaactttta acaagttctg tttgtcgttg agtcctgttg gtgctaattg taagtttgat 1920

gtagctgccc gtacaagaac caatgagcag gttgttagaa gtttgtatgt aatatatgaa 1980

gaaggagaca acatagtggg tgtaccgtct gataatagtg gtgtgcacga tttgtcagtg 2040

ctacacctag attcctgcac agattacaat atatatggta gaactggtgt tggtattatt 2100

agaaaaacta acaggacgct acttagtggc ttatattaca catcactatc aggtgatttg 2160

ttaggtttta aaaatgttag tgatggtgtc atctactctg taacgccatg tgatgtaagc 2220

gcacaagcag ctgttattga tggtaccata gttggggcta tcacttccat taacagtgaa 2280

ctgttaggtc taacacattg gacaacaaca cctaattttt attactactc tatatataat 2340

tacacaaatg ataggactcg tggcactgca attgacagta atgatgttga ttgtgaacct 2400

gtcataacct attctaacat aggtgtttgt aaaaatggtg cttttgtttt tattaacgtc 2460

acacattctg atggagacgt gcaaccaatt agcactggta atgtcacgat acctacaaac 2520

tttaccatat ccgtgcaagt cgaatatatt caggtttaca ctacaccagt gtcaatagac 2580

tgttcaagat atgtttgtaa tggtaaccct aggtgtaaca aattgttaac acaatacgtt 2640

tctgcatgtc aaactattga gcaagcactt gcaatgggtg ccagacttga aaacatggag 2700

gttgattcca tgttgtttgt ttctgaaaat gcccttaaat tggcatctgt tgaagcattc 2760

aatagttcag aaactttaga ccctatttac aaagaatggc ctaatatagg tggttcttgg 2820

ctagaaggtc taaaatacat acttccgtcc cataatagca aacgtaagta tcgttcagct 2880

atagaggact tgctttttga taaggttgta acatctggtt taggtacagt tgatgaagat 2940

tataaacgtt gtacaggtgg ttatgacata gctgacttag tatgtgctca atactataat 3000

ggcatcatgg tgctacctgg tgtggctaat gctgacaaaa tgactatgta cacagcatcc 3060

cttgcaggtg gtataacatt aggtgcactt ggtggaggcg ccgtggctat accttttgca 3120

gtagcagttc aggctagact taattatgtt gctctacaaa ctgatgtatt gaacaaaaac 3180

cagcagattc tggctagtgc tttcaatcaa gctattggta acattacaca gtcatttggt 3240

aaggttaatg atgctataca tcaaacatca cgaggtcttg ctactgttgc taaagcattg 3300

gcaaaagtgc aagatgttgt caacatacaa gggcaagctt taagccacct aacagtacaa 3360

ttgcaaaata atttccaagc cattagtagt tctattagtg acatttataa taggcttgac 3420

gaattgagtg ctgatgcaca agttgacagg ctgatcacag gaagacttac agcacttaat 3480

gcatttgtgt ctcagactct aaccagacaa gcggaggtta gggctagtag acaacttgcc 3540

aaagacaagg ttaatgaatg cgttaggtct cagtctcaga gattcggatt ctgtggtaat 3600

ggtacacatt tgttttcact cgcaaatgca gcaccaaatg gcatgatttt ctttcacaca 3660

gtgctattac caacggctta tgaaactgtg actgcttggc caggtatttg tgcttcagat 3720

ggtgatcgca cttttggact tgtcgttaaa gatgtccagt tgactttgtt tcgtaatcta 3780

gatgacaagt tctatttgac ccccagaact atgtatcagc ctagagttgc aactagttct 3840

gactttgttc aaattgaagg gtgcgatgtg ctgtttgtta atgcaactgt aagtgatttg 3900

cctagtatta tacctgatta tattgatatt aatcagactg ttcaagacat attagaaaat 3960

tttagaccaa attggactgt acctgagttg acatttgaca tttttaacgc aacctattta 4020

aacctgactg gtgaaattga tgacttagaa tttaggtcag aaaagctaca taacaccact 4080

gtagaacttg ccattctcat tgacaacatt aacaatacat tagtcaatct tgaatggctc 4140

aatagaattg aaacctatgt aaaatggcct tggtatgtgt ggctactaat aggcttagta 4200

gtaatatttt gcataccatt actgctattt tgctgttgta gtacaggttg ctgtggatgc 4260

ataggttgtt taggaagttg ttgtcactct atatgtagta gaagacaatt tgaaaattac 4320

gaaccaattg aaaaagtgca cgtccattaa 4350

<210> 2
<211> 606
<212> DNA
<213> PRRSV

<400> 2
atgagatgtt ctcacaaatt ggggcgtttc ttgactcctc actcttgctt ctggtggctt 60

tttttgctgt gtaccggctt gtcctggtcc tttgtcgctg gcagcggcag cagctcgaca 120

taccaataca tatataactt aacgatatgc gagctgaatg ggaccgactg gttgtccaac 180

cattttgatt gggcagtcga gacctttgtg ctttacccgg ttgccactca tatcctctca 240

ctgggttttc tcacaacaag ccattttttt gacgcgctcg gtctcggcgc tgtgtccact 300

acaggatttg ttggcgggcg gtatgtactc agcagcgtgt acggcgcttg tgctttcgca 360

gcgctcgtat gttttgtcat ccgtgctgtt aaaaattgca tggcttgccg ctatgcccac 420

acccggttta ccaacttcat tgtggacgac cgggggagaa tccatcggtg gaagtctcca 480

atagtggtag agaaattggg caaagctgaa gtcggtggcg accttgtcac catcaaacat 540

gtcgtcctcg aaggggttaa agctcaaccc ttgacgagga cttcggctga gcaatgggaa 600

gcctag 606

<210> 3
<211> 522
<212> DNA

<213>  PRRSV

<400>  3
atgggaagcc tagacgattt ttgcaatgat tctaccgccg cacaaaagct tgtgctagcc      60

tttagcatta catatacacc tataatgata tacgccctta aggtgtcacg cggccgactc     120

ctggggctgt tgcacatcct aatattcctg aattgttctt tcacattcgg atacatgaca     180

tatgtgcgtt ttcaatccac caaccgtgtc gcacttactc tgggggctgt tgtcgccctt     240

ctgtggggtg tttacagctt cacagagtca tggaagtttg ttacttccag atgcagattg     300

tgttgcctag gccggcgata cattctggcc cctgcccatc acgtagaaag tgctgcaggt     360

ctccattcaa tcccagcgtc tggtaaccga gcatacgctg tgagaaagcc cggactaaca     420

tcagtgaacg gcactctagt tccaggactt cggagcctcg tgctgggcgg caaacgagct     480

gttaaacgag gagtggttaa cctcgtcaag tatggccggt aa                        522


<210>  4
<211>  37
<212>  DNA
<213>  Artificial Sequence
<220>
<223>  oligonucleotide primer

<400>  4
aacaggtcct accatgagat gttctcacaa attgggg                               37


<210>  5
<211>  38
<212>  DNA
<213>  Artificial Sequence

<220>
<223> oligonucleotide primer

<400> 5
ccgctaagcc taggcttccc attgctcagc cgaagtcc 38

<210> 6
<211> 77
<212> DNA
<213> Artificial Sequence
<220>
<223> oligonucleotide primer

<400> 6
cggctgagca atgggaagcc tagaaaatta ttacatatgg tataactaaa caaaatggga 60

agcctagacg attttg 77

<210> 7
<211> 21
<212> DNA
<213> Artificial Sequence
<220>
<223> oligonucleotide primer

<400> 7
gccggcctag acaacacaat c 21

<210> 8
<211> 21
<212> DNA
<213> Artificial Sequence
<220>
<223> oligonucleotide primer

<400> 8
gattgtgttg tctaggccgg c 21

<210>  9
<211>  29
<212>  DNA
<213>  Artificial Sequence
<220>
<223>  oligonucleotide primer

<400>  9
gctaagctta ccggccatac ttgacgagg                                          29

<210>  10
<211>  7
<212>  PRT
<213>  PRRSV

<400>  10

Thr Tyr Gln Tyr Ile Tyr Asn
1               5

<210>  11
<211>  20
<212>  PRT
<213>  Sus scrofa

<400>  11

Arg Gly Gln Gly Ser Met Asp Glu Gly Thr Ala Asp Glu Arg Ala Pro
1               5                   10                  15

Leu Ile Arg Thr
            20

<210>  12
<211>  60
<212>  DNA
<213>  Sus scrofa

<400>  12
agaggacagg gatccatgga tgagggaact gcagacgaac gagcacccct catacgaacc      60

<210>  13
<211>  564
<212>  DNA
<213>  Artificial Sequence
<220>

<223>  PRRSV epitope ORF5-ORF6

<400>  13
tcgacatacc aatacatata taacttaacg atatgcgagc tgatgggaag cctagacgat      60

ttttgcaatg attctaccgc cgcacaaaag cttgtgctag cctttagcat tacatataca     120

cctataatga tatacgccct taaggtgtca cgcggccgac tcctggggct gttgcacatc     180

ctaatattcc tgaattgttc tttcacattc ggatacatga catatgtgcg ttttcaatcc     240

accaaccgtg tcgcacttac tctgggggct gttgtcgccc ttctgtgggg tgtttacagc     300

ttcacagagt catggaagtt tgttacttcc agatgcagat tgtgttgcct aggccggcga     360

tacattctgg cccctgccca tcacgtagaa agtgctgcag gtctccattc aatcccagcg     420

tctggtaacc gagcatacgc tgtgagaaag cccggactaa catcagtgaa cggcactcta     480

gttccaggac ttcggagcct cgtgctgggc ggcaaacgag ctgttaaacg aggagtggtt     540

aacctcgtca agtatggccg gtaa                                            564

<210>  14
<211>  624
<212>  DNA
<213>  Artificial Sequence
<220>

<223>  Epitope ORF5_ORF6_LIMP-II

<400>  14
tcgacatacc aatacatata taacttaacg atatgcgagc tgatgggaag cctagacgat      60

ttttgcaatg attctaccgc cgcacaaaag cttgtgctag cctttagcat tacatataca     120

cctataatga tatacgccct taaggtgtca cgcggccgac tcctggggct gttgcacatc     180

ctaatattcc tgaattgttc tttcacattc ggatacatga catatgtgcg ttttcaatcc     240

accaaccgtg tcgcacttac tctgggggct gttgtcgccc ttctgtgggg tgtttacagc     300

ttcacagagt catggaagtt tgttacttcc agatgcagat tgtgttgcct aggccggcga     360

tacattctgg cccctgccca tcacgtagaa agtgctgcag gtctccattc aatcccagcg     420

tctggtaacc gagcatacgc tgtgagaaag cccggactaa catcagtgaa cggcactcta     480

gttccaggac ttcggagcct cgtgctgggc ggcaaacgag ctgttaaacg aggagtggtt     540

aacctcgtca agtatggccg gagaggacag ggatccatgg atgagggaac tgcagacgaa     600

cgagcacccc tcatacgaac ctaa                                            624


<210>  15
<211>  5
<212>  PRT
<213>  Artificial Sequence
<220>
<223>  peptide linker

<400>  15

Gly Gly Pro Gly Gly
1               5